# EUROPEAN PATENT APPLICATION

(11) **EP 1 580 260 A1**
(43) Date of publication of application: **28.09.2005**
(21) Application number: 03780837.5
(22) Date of filing: 17.12.2003
(51) Int. Cl.: C12M 1/26

(54) **CELL SEPARATION AND COLLECTION APPARATUS AND SEPARATION AND COLLECTION METHOD**

(30) Priority: 25.12.2002 JP 2002375439
(71) Applicant: National Institute of Advanced Industrial Science and Technology, Tokyo 100-8921 (JP)
(72) Inventor: KANAMORI, Toshiyuki, c/o Nat. Inst. of Advanced I., Tsukuba-shi, Ibaraki 305-8565 (JP); ITAYAGOSHI, Midori, c/o Nat. Inst. of Advanced In., Tsukuba-shi, Ibaraki 305-8565 (JP); YAMAGUCHI, Manae, c/o Nat. Inst. of Advanced Ind., Tsukuba-shi, Ibaraki 305-8565 (JP); SUMARU, Kimio, c/o Nat. Inst. of Advanced Ind. Sce, Tsukuba-shi, Ibaraki 305-8565 (JP); SHINBO, Toshio, c/o Nat. Inst. of Advanced Ind Sce, Tsukuba-shi, Ibaraki 305-8565 (JP); OU, Hekisho, c/o Nat. Inst. of Advanced Ind. Sce., Tsukuba-shi, Ibaraki 305-8566 (JP)
(74) Representative: Zimmermann, Gerd Heinrich
(86) International application number: PCT/JP2003/016170
(87) International publication number: WO 2004/058936

(57) **Abstract**

The present invention provides a cell separation and recovery apparatus and a separation and recovery method, by which target cells are separated and recovered from a cell source in a large amount within a short period of time without inflicting damage the cells. The cell separation and recovery apparatus 10 of the present invention is **characterized in that** it is equipped with a treatment part 11 having a non-woven fabric bound with a polymer capable of showing a hydrophobic nature at a temperature higher than a predetermined temperature and a hydrophilic nature at a temperature lower than the predetermined temperature and a physiologically active substance capable of binding to the target cells, and a liquid temperature controlling part 14 which controls liquid temperature of the treatment part 11, and is constructed so as to release the cells captured on the non-woven fabric and recover them from the non-woven fabric by changing the liquid temperature of the treatment part around the predetermined temperature by the liquid temperature controlling part.

## Description

### Technical Field

The present invention relates to a cell separation and recovery apparatus and a separation and recovery method, by which target cells are separated and recovered by using a polymer capable of changing its hydrophilic property and the like, depending on temperature. It is possible to apply the present invention to, for example, a case in which predetermined cells are separated and recovered in bio-tissue engineering, cell therapy and the like.

### Background Art

In the current frontier medicine, techniques which use mammalian cells, such as gene therapy, hybrid type artificial organs or bio-tissue engineering, are occupying the attention of the world. Such techniques are based on the rapid progress of molecular biology, and the development of flow cytometry which can separate specific cells alone from a cell suspension at high accuracy can be exemplified as one of their important technical basis.

However, since the flow cytometry was developed originally for use in studies on molecular biology, its separation accuracy is considerably high, but its treating rate is at the test tube level. Thus, it cannot be said that this is suited for clinical use which frequently has a case of requiring a large number of cells within a markedly short period of time. For example, when a hybrid type liver is applied to a patient of fluminant hepatitis whose life-saving ratio is said to be about 30%, a predetermined amount (e.g., from several hundred mg to several kg) of hepatocytes must be prepared within several days at the latest. Similarly, in the case of artificial skin for saving a patient of serious burn injury, it is necessary to culture a required amount of cells of the patient within several days to several weeks.

Thus, in order to realize a medical treatment using mammalian cells in the clinical field, a cell separation and recovery apparatus corresponding to the purpose from a cell source within a short period of time (cell separator) is necessary and indispensable.

Up to now, various methods and apparatuses have been proposed regarding cell separation, for example, as can be seen in the following patent references (1) to (17).
(1) JP-A-8-201384
(2) JP-A-9-75450
(3) JP-A-10-14565
(4) JP-A-11-56351
(5) JP-A-11-290060
(6) JP-A-2000-166541
(7) JP-A-2001-136956
(8) JP-A-2001-17161812
(9) JP-A-2001-161352
(10) JP-A-2001-178
(11) JP-A-2001-78757
(12) JP-A-2002-80377
(13) JP-A-2002-87971
(14) JP-A-7-265407
(15) JP-A-9-108334
(16) JP-A-10-137557
(17) JP-A-2000-83649

By the way, a technique in which a physiologically active substance (ligand) is physically adhered to the bottom face of a plastic cell culture container (culture dish), and a cell having a receptor which specifically binds to the physiologically active substance is captured, is used in the field of cell engineering and molecular biology. However, when a protein such as immunoglobulin is used as the ligand, not only the compatibility (affinity) between the ligand and the receptor is considerably strong, but also physical adsorption between the ligand and the culture dish bottom is also strong, so that it is difficult in general to recover cells from the culture dish bottom.

Thus, an affinity column has been developed, in which an antibody, a hormone or a protein such as a lectin is immobilized on the surface of polysaccharide beads having a diameter of several ten to several hundred µm, specified cells are captured thereby through affinity adsorption, and the cells are recovered with an eluent. In addition, a technique has also been developed, in which cells are recovered by the similar mechanism of a column using nylon fibers in which a physiologically active substance is physically adsorbed or covalently bound to the surface.

However, in these techniques, it is necessary to shift the pH and salt concentration of the eluent extremely from the physiological conditions, and the influence caused thereby upon the cells to be recovered cannot be disregarded. In addition, the column cannot set the flow rate to a high level because of the large pressure loss, and the fiber cannot increase surface area per unit volume, so that further improvement is necessary in each case in terms of the treating quantity per unit time.

Accordingly, a technique (cell manipulation) in which poly(N-isopropylacrylamide) is graft-polymerized on the bottom face of a culture dish by electron beam, cells and a medium are put into it to carry out culturing at 37°C, and then the temperature is lowered to 30°C to recover the cells as a sheet has been developed (e.g., see T. *Okano et al., J. Biomed. Mater. Res.,* 27, 1243 (1993)).

Since the poly(N-isopropylacrylamide) has a lower critical solution temperature (LCST) of about 32°C, it becomes hydrophobic at 37°C and hydrophilic at 32°C or less. In addition, since cells are not adhered to the hydrophilic surface in general, cells cultured into a sheet form are spontaneously peeled off by culturing the cells at a temperature higher than the LCST and then cooling them to a temperature lower than the LCST. Recovering of such a sheet-like cultured product has rendered possible reproduction of a phenomenon in which cells act in harmony, such as synchronous pulsation of myocardial cells.

In addition, studies have also been carried out on materials whose water permeability changes at their lower critical solution temperature, by introducing poly(N-isopropylacrylamide) to the fiber surface of a polypropylene non-woven fabric using plasma post-polymerization (e.g., see S.Y. Kim *et al., J. Appl. Polym. Sci.,* **84****,** 1168 (2002)).

### Disclosure of the Invention

In the tissue engineering, cell therapy and the like, concern has been directed toward the development of a method for separating and recovering cells corresponding to the purpose from a cell source in a large amount within a short period of time without damaging the cells.

The present invention has been made based on such a technical problem, and its object is to provide a cell separation and recovery apparatus and a method for separating and recovering cells, in which cells corresponding to the purpose are separated and recovered from a cell source in a large amount within a short period of time without damaging the cells.

In order to attain the above-described object, the inventors of the present invention have conducted studies on the use of a thermosensitive polymer whose hydrophilic and hydrophobic properties change, depending on temperature, by paying attention to a cell manipulation technique which uses interaction between the surface of a polymer and the surface of cells. As a result, the present invention has been accomplished by developing a cell separation recovery technique which recovers specified cells non-invasively.

In order to attain the above-described object, the present invention provides a cell separation and recovery apparatus, which comprises a treatment part having a non-woven fabric which is bound with a polymer showing a hydrophobic nature at a temperature higher than a predetermined temperature and showing a hydrophilic nature at a temperature lower than the predetermined temperature and a physiologically active substance capable of binding to target cells, and a liquid temperature controlling part for controlling liquid temperature of the treatment part, wherein the cells captured on the non-woven fabric are released and recovered from the non-woven fabric by changing the liquid temperature of the treatment part around the prescribed temperature with the liquid temperature controlling part.

In the separation and recovery apparatus of the present invention, the physiologically active substance may be bound to the non-woven fabric via the polymer.

Also, the physiologically active substance may be directly bound to the non-woven fabric.

Also, the above-described physiologically active substance may be bound to the non-woven fabric via a spacer.

Also, the separation and recovery apparatus of the present invention may be constituted so as to capture the cells on the non-woven fabric captures when the liquid temperature is lower than the predetermined temperature, and to release the captured cells from the non-woven fabric when the liquid temperature is higher than the predetermined temperature.

Also, the separation and recovery apparatus may be constituted so as to capture the cells on the non-woven fabric captures when the liquid temperature is higher than the predetermined temperature, and to release the captured cells from the non-woven fabric when the liquid temperature is lower than the predetermined temperature.

Also, in the separation and recovery apparatus of the present invention, the polymer is preferably poly(N-isopropylacrylamide).

Also, the physiologically active substance is preferably at least one selected from the group consisting of an antigen, an antibody and a protein such as a fragment thereof.

Also, the present invention provides a method for separating and recovering cells, which comprises:
bringing a liquid containing cells into contact with a non-woven fabric which is bound with a polymer showing a hydrophobic nature at a temperature higher than a predetermined temperature and showing a hydrophilic nature at a temperature lower than the predetermined temperature and a physiologically active substance capable of binding to target cells, to thereby capture target cells on the non-woven fabric for separating the cells,
releasing the captured cells from the non-woven fabric by changing the temperature of the non-woven fabric around the predetermined temperature, and
recovering the cells released from the non-woven fabric.

Also, the present invention provides a method for separating and recovering cells, which comprises:
bringing a liquid containing cells into contact with the non-woven fabric in the treatment part using the separation and recovery apparatus of the present invention to capture target cells on the non-woven fabric for separating the cells from the liquid,
releasing the captured cells from the non-woven fabric by changing the liquid temperature of the treatment part around the predetermined temperature, and
recovering the cells released from the non-woven fabric.

### Brief Description of the Drawings

Fig. 1 is a schematic illustration explaining a formation method of a non-woven fabric for separating cells in the present invention.
Fig. 2 is a schematic illustration explaining conditions for separating and recovering cells in the present invention.
Fig. 3 is a schematic illustration explaining conditions for recovering cells in the embodiment 2.
Fig. 4 is a schematic illustration explaining conditions for recovering cells in the embodiment 3.
Fig. 5 is an explanatory drawing showing an example of the cell separation and recovery apparatus.
Fig. 6 is a graph showing a result of a graft yield of polyNIP AAm based on the reaction time.
Fig. 7 is a graph showing a result of a graft yield based on the pore size of a polyNIPAAm-g-PP non-woven fabric.
Fig. 8 is a graph showing a result of water permeation based on temperature.
Fig. 9 is a graph showing a relationship between the antibody liquid concentration and the number of cells.
Fig. 10 is a graph showing a relationship between the culturing period and the number of cells.
Fig. 11 is a graph showing the number of cells captured by a non-woven fabric made of polyNIP AAm-g-PP.

### Best Mode for Carrying Out the Invention

### Embodiment 1:

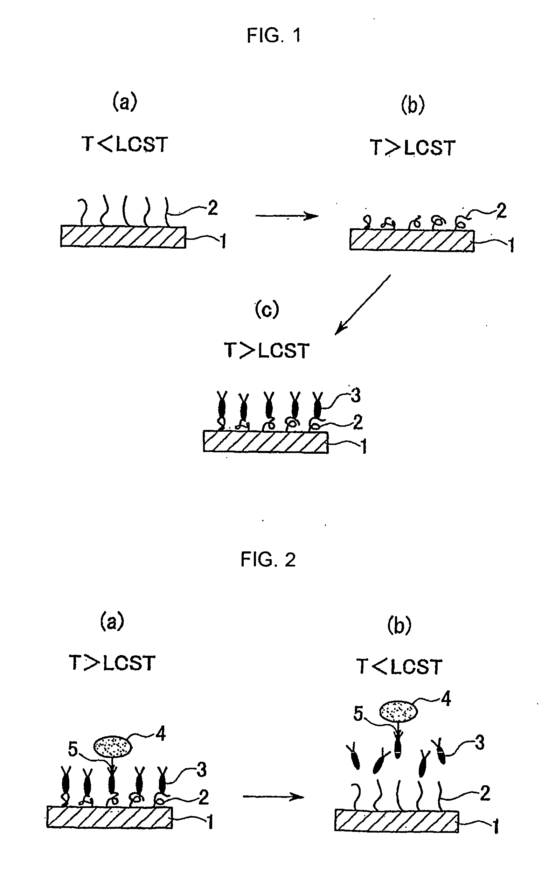

Fig. 1 is a schematic illustration explaining a formation method of a non-woven fabric for separating cells in the present invention.

Fig. 1(a) illustrates a non-woven fabric 1 to which a polymer 2 is grafted in water having a temperature lower than the lower critical solution temperature (hereinafter referred to as "LCST") of the polymer 2. The polymer 2 is composed of a thermosensitive polymer, poly(N-isopropylacrylamide) (hereinafter referred to as "polyNIP AAm").

The kind of the fiber which constitutes the non-woven fabric 1 can be optionally selected from synthetic fibers, natural fibers and the like. According to the present invention, it is preferable to use a non-woven fabric formed from a polypropylene (hereinafter referred to as "PP") fiber. In addition, it is preferable that pores having an equivalent diameter of 5 to 30 µm are present in the non-woven fabric 1 at a porosity of 80% or more. Since such a non-woven fabric has a markedly large fiber surface area per unit volume and considerably low permeation resistance (pressure loss) of cell suspension, it can be used particularly preferably as the material for cell separation of a cell separation and recovery apparatus (cell separator apparatus).

The thermosensitive polymer polyNIP AAm has a characteristic in that its hydrophilic and hydrophobic properties change around the lower critical solution temperature. For example, polyNIP AAm shows a hydrophobic property at a temperature higher than its LCST, and a hydrophilic property at a temperature lower than its LCST, around the LCST which is about 32°C. In Fig. 1(a), the temperature of water in which the non-woven fabric 1 is soaked is lower than the LCST of polymer 2, so that the polymer 2 shows a hydrophilic property and is stretched in water. In this connection, although polyNIP AAm was used as the polymer 2 in this example, a thermosensitive polymer other than this may also be used.

As the method for binding the polymer 2 to the fiber surface of the non-woven fabric 1, for example, a method in which the NIP AAm monomer is graft-polymerized on the surface of the non-woven fabric 1 by a plasma post-polymerization method can be employed. Specifically, plasma irradiation to the non-woven fabric 1 is carried out in an atmosphere of argon, and then the NIP AAm monomer is brought into contact therewith for graft polymerization to the fiber surface of the non-woven fabric 1. Thus, the non-woven fabric 1 is modified by the polymer 2 composed of polyNIP AAm, and its hydrophilic degree is changed depending on temperature. Accordingly, it becomes possible to bind to a physiologically active substance at a predetermined temperature or higher, and to release it at the temperature or lower. The physiologically active substance is not limited to the antibody 3, and various substances which specifically bind to target cells, such as an antigen, an antibody, CD4; CD8, CD86, CD80, CD34 and IgG antibodies thereof, can be used and an appropriate physiologically active substance can be selected from these substances, depending on the target cells, wherein at least one selected from the group consisting of an antigen, an antibody and a fragment thereof can be exemplified as the preferred physiologically active substance.

When the water temperature is increased to a level higher than the LCST, the polymer 2 shows a hydrophobic property and is transformed from the state shown in Fig. 1(a) into the state shown in Fig. 1(b). Specifically, when the polymer 2 chain shows a hydrophobic property, it repels with the hydrophilic water molecule and shrinks to form a rounded state as shown in Fig. 1(b). As shown in Fig. 1(c), the antibody 3 as a physiologically active substance is bound to the polymer 2 on the non-woven fabric 1 of this state via a hydrophobic bond. In this case, the antibody 3 is, for example, an IgG. The non-woven fabric 1 obtained in the above manner can adsorb and release the antibody 3 as a physiologically active substance.

The non-woven fabric 1 obtained in the above manner has a property of varying the hydrophilic degree depending on the temperature. Thus, a specified physiologically active substance can be bound via a hydrophobic bond or a hydrophilic bond at a predetermined temperature, and the bond can be separated at a temperature different from the above-described predetermined temperature. Accordingly, by making use of this property, the predetermined cells bound to the physiologically active substance can be separated and recovered by changing the temperature after the binding of cells while using the non-woven fabric 1 bound to a physiologically active substance having a property to specifically bind to predetermined cells,.

Fig. 2 is a schematic illustration explaining conditions for separating and recovering cells in the present invention.

The polymer 2 composed of polyNIP AAm is bound to the surface of the non-woven fabric 1, and the polymer 2 and antibody 3 are bound via a hydrophobic bond at a liquid temperature higher than the LCST. By spreading thereto a cell suspension (mixed liquid) containing a cell 4 having a surface antigen 5 capable of recognizing the antibody 3, the surface antigen 5 and antibody 3 are subjected to antigen-antibody reaction as shown in Fig. 2(a). In this case, when the antibody 3 is an IgG, a CD3 expressing stem cell can, for example, be used as the cell 4.

In this connection, the cell 4 and antibody 3 are bound to each other at one point (one position) in Fig. 2(a), but they are frequently bound at multiple points in reality. The cell 4 bound in this manner can be cultured. In this case, a cell having a surface antigen which does not recognize the antibody 3 does not bind to the antibody 3. In order to remove unnecessary cells which are not bound to the antibody 3, the cell suspension is washed with a buffer or the like. In this connection, when the antibody is an IgG, the first temperature is preferably from 35°C to 39°C, more preferably 37°C.

Next, the cell 4 and antibody 3 are released from the non-woven fabric 1 to which the polymer 2 composed of polyNIP AAm is bound, by lowering the liquid temperature than the LCST. Specifically, by lowering the temperature than the LCST, the surface of the PP non-woven fabric 1 introduced with the polymer 2 is changed to a hydrophilic nature. As a result, as shown in Fig. 2(b), the hydrophobic bond between the polymer 2 and antibody 3 is removed so that the target cell 4 can be recovered under a state of being bound to the antibody 3. In this connection, when the antibody is an IgG, the second temperature is preferably from 2°C to 6°C, more preferably 4°C.

When the non-woven fabric 1 which can adsorb and release a physiologically active substance (antibody 3) is used in the above manner, the target cells can be separated from other cells within a short period of time and recovered in a large amount without causing damage.

### Embodiment 2:

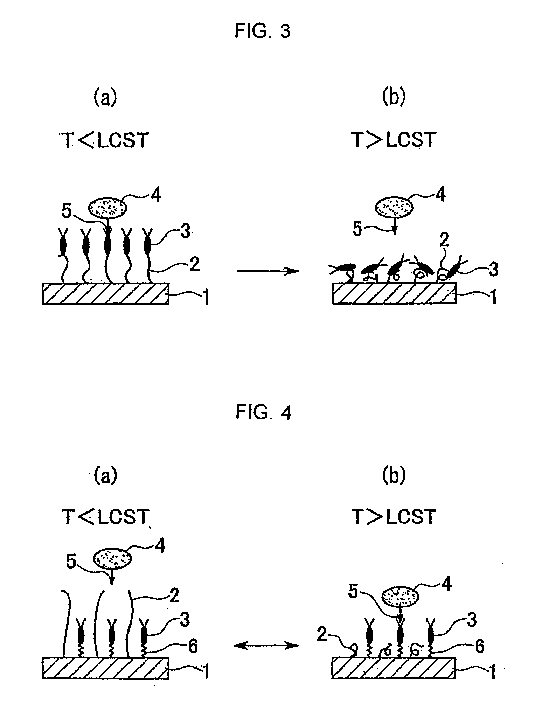

Fig. 3 is a schematic illustration explaining conditions for recovering cells in the embodiment 2.

According to this embodiment 2, separation and recovery of cells can be carried out in almost the same manner as in the above-described method shown in Fig. 1, except that the treating temperature conditions were changed. The non-woven fabric 1 to be used herein has a structure in which the polymer 2 composed of polyNIPAAm is bound to its surface, and the antibody 3 is bound as a physiologically active substance to the polymer 2, and in this non-woven fabric 1, the polymer 2 and antibody 3 are bound to each other via a covalent bond.

According to the embodiment 2, as shown in Fig. 3(a), the surface antigen 5 of the cell 4 and the antibody 3 are bound to each other at a liquid temperature lower than the LCST. By providing the polymer 2 with a hydrophobic property at a liquid temperature higher than the LCST, the bond between the surface antigen 5 and antibody 3 is separated and the cell 4 is recovered. In this case, the higher-order structure of the polymer 2 is changed by temperature, thereby effecting exposure of the antibody 3 at a liquid temperature lower than the LCST and concealment the antibody 3 at a liquid temperature higher than the LCST, so that capture and release of the cell 4 can be carried out. In this way, in the embodiment 2, the temperature and process are reversed from the method of the embodiment 1, so that the cell must be cultured at a temperature lower than the LCST, but being a structure in which the polymer 2 and antibody 3 are strongly bound to each other via a covalent bond, the cell 4 can be kept more effectively.

### Embodiment 3:

Fig. 4 is a schematic illustration explaining conditions for recovering cells in the embodiment 3.

In the embodiment 3, the polymer 2 composed of polyNIP AAm is bound onto the non-woven fabric 1 in the same manner as in the embodiment 1. As shown in Fig. 4(a), it has a structure in which the antibody 3 is bound to the surface of non-woven fabric 1, not via the polymer 2 but via a spacer 6. In this case, the spacer 6 functions for keeping the antibody 3 on the non-woven fabric 1, and is, for example, polyethylene glycol. According to this structure, it is preferable to design it in such a manner that the length from the non-woven fabric 1 to the antibody 3 via the spacer 6 is shorter than the length of polymer 2 elongating from the non-woven fabric 2. Also, the antibody may be directly bound to the non-woven fabric 1 without the spacer. In addition, it is preferable that the polymer 2 is polymerized to the non-woven fabric 1 at an optionally set density.

When the polymer 2 composed of polyNIP AAm is provided with a hydrophobic property by setting the liquid temperature to a level higher than the LCST, the polymer 2 shrinks as shown in Fig. 4(b), and as a result, the surface antigen 4 of the cell 4 binds to the antibody 3. Alternatively, when the liquid temperature is set to a level lower than the LCST, the polymer 2 stretches as shown in Fig. 4(a), and the bond between the surface antigen 5 of the cell 4 and the antibody 3 is separated, so that it becomes possible to recover the cell 4. In this embodiment 3, the polymer 2 acts only when the captured cell 4 is recovered, so that the period of time for lowering the temperature than the LCST is short, and the cell 4 which is hardly influenced by temperature can be recovered more efficiently.

The present invention also provides a cell separation and recovery apparatus, which can carry out the above-described embodiments 1 to 3.

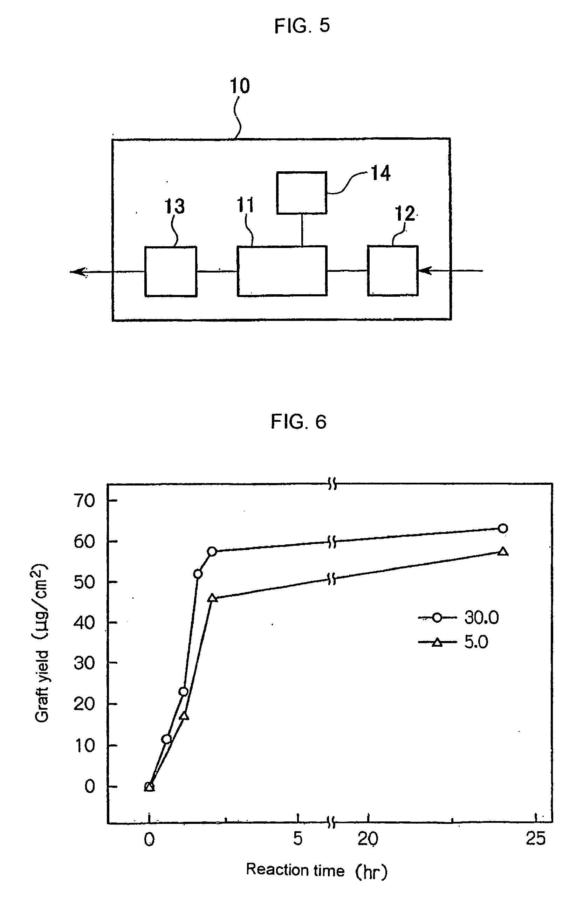

Fig. 5 is an explanatory drawing showing an example of the cell separation and recovery apparatus of the present invention.

The cell separation and recovery apparatus 10 shown in Fig. 5 is equipped with a treatment part 11 having the non-woven fabric 1 to which the polymer 2 composed of polyNIP AAm and a physiologically active substance, such as the antigen 3, are bound, which is used in the embodiments 1 to 3, a liquid introducing part 12 which introduces a cell-containing liquid into the treatment part 11, a liquid discharging part 13 which discharges the liquid from the treatment part 11 and a liquid temperature controlling part 14 which controls liquid temperature in the treatment part 11, and is constructed in such a manner that the cells captured by the above-described non-woven fabric 1 are released and recovered from the non-woven fabric 1 by changing the liquid temperature of the treatment part 11 around the LCST by the liquid temperature controlling part 14. In this connection, the cell separation and recovery apparatus 10 can be equipped with a regulating part which regulates the liquid temperature controlling part 14 and other necessary constructions.

This treatment part 11 will be enough when it can keep the non-woven fabric 1 and bring it into contact with a cell-containing liquid or the like liquid, so that its specific structure, shape, size and the like are not limited, and treating vessels or tanks having various sizes can be used depending on the introducing amount of the liquid to be treated. In order to accelerate the adsorption and release of cells and also to unify the liquid temperature inside thereof, it is preferable to arrange an appropriate stirring device to this treatment part 11. It is preferable that the non-woven fabric 1 arranged in the treatment part 11 is attached to the treatment part 11 in a detachable manner.

Also, each of the above-described liquid introducing part 12 and liquid discharging part 13 can be constructed by connecting an appropriate pipe line or the like to the treatment part 11, depending on the structure, shape, size and the like of the treatment part 11, and a liquid storing part, a bulb, a branch pipeline, a flow meter and the like can be arranged as occasion demands.

The above-described liquid temperature controlling part 14 has a temperature controlling means, for example, at least one of a electric heater and a cooler, for changing the liquid temperature in the treatment part 11 around a predetermined temperature (namely LCST of the polymer 2), and preferably has a structure which can regulate sending of electricity to the temperature controlling means so that the inside of the treating vessel 11 becomes a set temperature, by comparing temperature measuring data from a temperature measuring means, such as a temperature sensor, arranged in the treatment part 11 with the set temperature.

According to this cell separation and recovery apparatus 10, the captured cells can be released from the non-woven fabric 1 by arranging the non-woven fabric 1 in the treatment part 11, introducing a liquid containing the target cells continuously or intermittently into the treatment part 11 through the liquid introducing part 12, allowing the non-woven fabric 1 to specifically capture the target cells alone and thereby separating them from the other cells, and then changing the liquid temperature of the treatment part 11 around the LCST, so that a large amount of the target cells can be recovered via the liquid discharging part 13 at high efficiency without injuring them. Regarding the principle of capture of cells to the non-woven fabric 1 and release of the captured cells therefrom, any one of the methods of the above-described embodiments 1 to 3 can be employed.

In this connection, it is needless to say that the constructions exemplified in the above-described embodiments can be freely selected or optionally changed to other constructions, without overstepping the gist of the present invention.

### Examples.

### Preparation of PP non-woven fabric in which polyNIP AAm is graft-polymerized on the fiber surface:

A PP non-woven fabric having an average pore size of 5, 10 or 30 µm was used as the base material. In an atmosphere of argon, plasma irradiation to this non-woven fabric was carried out for 1 minutes at an output of 10 W. After the plasma irradiation, an aqueous NIP AAm monomer solution (1 to 10 wt %) was put into a reaction container. Thereafter, the reaction container was put into a shaking bath of 60°C, and graft polymerization was carried out. The polymerization was confirmed by using a total reflection type Fourier transform infrared spectrophotometry (ATR/FT-IR). The graft yield of the PP non-woven fabric prepared by introducing polyNIP AAm into the fiber surface (hereinafter referred to as "polyNIP AAm-g-PP non-woven fabric") was calculated from the change in weight of the non-woven fabric before and after the polymerization.

The permeated amount of water through the polyNIP AAm-g-PP non-woven fabric or unmodified PP non-woven fabric was measured using a permeation cell device. The effective membrane area of the non-woven fabric used in the measurement was 17.3 cm². The change in the permeation amount by temperature was examined by measuring permeated amount of water at a temperature higher than or lower than the LCST of polyNIP AAm.

### Capture and detachment of antibody by temperature change:

The polyNIP AAm-g-PP non-woven fabric cut out into a diameter of 1.5 cm was incubated at 37°C for 3 hours in Dulbecco's phosphate-buffered saline (PBS) containing fluorescein isothiocyanate-labeled IgG (FITC-IgG) (50 µg/ml) (hereinafter referred to as "antibody solution"), and the state of capturing of IgG was observed under a fluorescence microscope.

Next, 1 ml of PBS was added thereto and exposed to 4°C for 15 minutes. Thereafter, the state of the polyNIPAAm-g-PP non-woven fabric was again observed under the fluorescence microscope.

### Result: Preparation of polyNIP AAm-g-PP non-woven fabric:

Fig. 6 is a graph showing a result of the graft yield of polyNIPAAm based on the reaction time. As shown in Fig. 6, the graft yield of polyNIP AAm increased along with the increase of polymerization reaction time. In addition, the graft yield of polyNIP AAm was high when the monomer concentration was high.

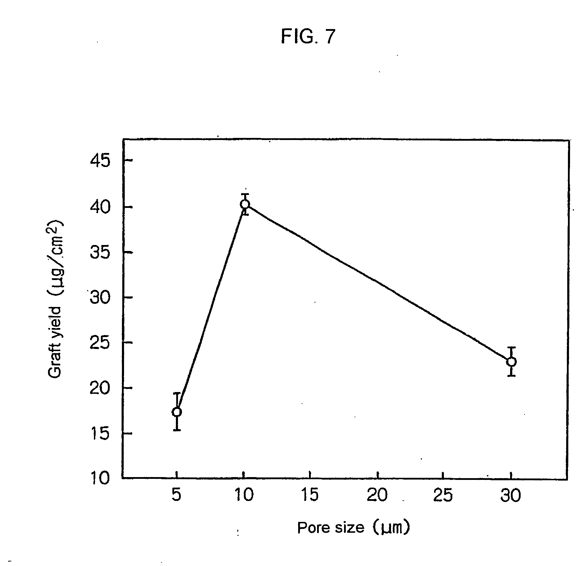

Fig. 7 is a graph showing a result of the graft yield based on the pore size of polyNIP AAm-g-PP non-woven fabric. As shown in Fig. 7, the average pore size of the polyNIP AAm-g-PP non-woven fabric also exerted influence on the graft yield.

### Result: Permeation of water through polyNIP AAm-g-PP non-woven fabric:

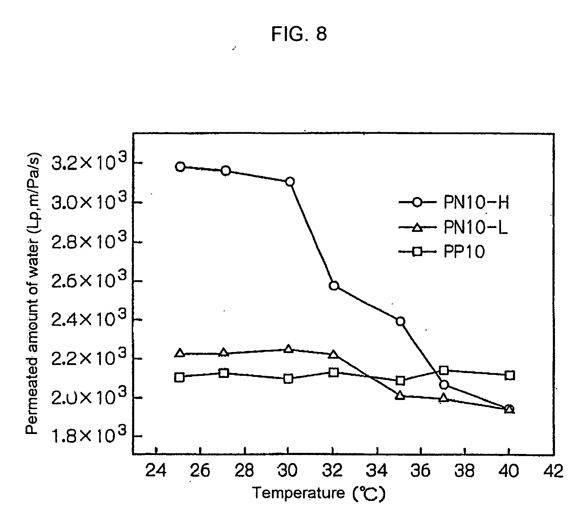

Influence of temperature on the permeated water (Lp) through polyNIP AAm-g-PP non-woven fabric was observed. Fig. 8 is a graph showing a result of water permeation based on temperature. As shown in Fig. 8, the permeated amount of water through the polyNIP AAm-g-PP non-woven fabric at a temperature lower than the LCST was larger than the case of an unmodified PP non-woven fabric. Also, at a temperature higher than the LCST, the permeated amount of water through the polyNIP AAm-g-PP non-woven fabric was reduced with the increase of temperature. It was considered that not only change in the pore size by polymerization but also microscopic change in the hydrophobic property on the surface have influence on the water permeation through the polyNIP AAm-g-PP non-woven fabric.

### Result: Capturing and detachment of antibody on the surface of polyNIP AAm-g-PP non-woven fabric:

States of capturing and detachment of FITC-labeled IgG by and from the polyNIP AAm-g-PP non-woven fabric and capturing and detachment of FITC-labeled IgG by and from the polyNIP AAm-g-PP non-woven fabric were observed under a fluorescence microscope. At a temperature higher than the LCST (37°C), the polyNIP AAm-g-PP non-woven fabric captured the FITC-IgG.

When the temperature was changed to a level lower than the LCST, the captured FITC-IgG was detached from the polyNIP AAm-g-PP non-woven fabric. The same test was carried out on the unmodified PP non-woven fabric, too. The antibody solution easily permeated into the polyNIP AAm-g-PP non-woven fabric, but hardly permeated into the unmodified PP non-woven fabric. The antibody was captured in a large amount only by a part where the antibody solution permeated, but the antibody was not detached when the temperature was changed to a level lower than the LCST.

Based on the above results, it was suggested that the fiber surface of the polyNIP AAm-g-PP non-woven fabric became hydrophobic at 37°C, namely a temperature higher than the LCST of the polyNIP AAm-g-PP non-woven fabric, and the FITC-IgG was captured by the fiber surface via a hydrophobic bond. In addition, when the temperature was lowered than the LCST, the fiber surface of the polyNIP AAm-g-PP non-woven fabric became hydrophilic, and the FITC-IgG was detached. Thus, although not all of the molecules captured, but the FITC-IgG was detached from the non-woven fabric when the temperature was lowered from 37°C to 4°C. Based on these results, cells bound to IgG can be separated and recovered together with IgG, for example, by binding IgG-bound cells to the IgG captured by the polyNIP AAm-g-PP non-woven fabric, and changing the temperature to a level of lower than the LCST.

### Cell capture accelerating effect of antibody solution concentration:

A piece of the polyNIP AAm-g-PP non-woven fabric having a diameter of 1.5 cm was put into each of 10 wells of a 24 well plate, and held with a glass ring to prevent its floating.

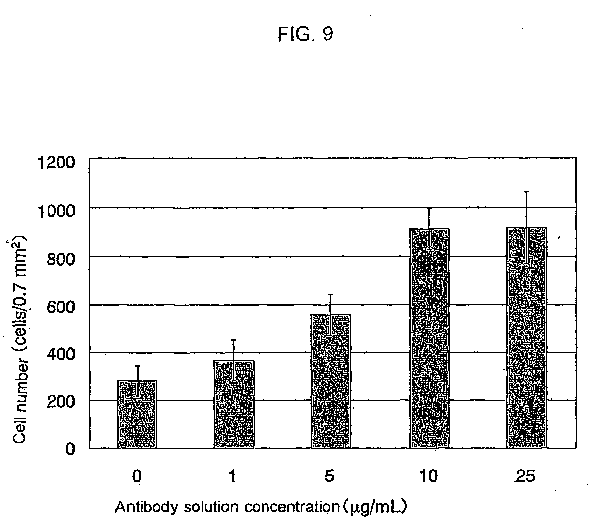

Dulbecco's phosphate-buffered saline (PBS) containing 0, 1, 5, 10 or 25 µg/ml in concentration of an anti-mouse CD80 monoclonal antibody (hereinafter referred to as "antibody solution") was added to each well and incubated at 37°C for 8 hours or more. Next, the polyNIP AAm-g-PP non-woven fabric was washed twice with PBS of 37°C. After 5×10⁵/ml CD80 hybridoma suspension suspended in RPMI-1640 medium (Sigma Aldrich Japan) was prepared, the suspension was added at 1 ml to respective wells and incubated at 37°C for 60 minutes. After washing these pieces of the polyNIP AAm-g-PP non-woven fabric twice with PBS, methanol fixation and Giemsa staining were carried out, and the number of captured cells was counted by observing under a microscope. Antibody concentration-dependent cell adsorption accelerating effect of the CD80 hybridoma cell was observed (Fig. 9). Significant cell capturing ability was observed particularly at an antibody concentration of 10 µg/ml or more.

### Selective capture of cells by monoclonal antibody and its periodical change:

A piece of the polyNIP AAm-g-PP non-woven fabric having a diameter of 1.5 cm was put into each of 8 wells of a 24 well plate, and held with a glass ring to prevent its floating.

Dulbecco's phosphate-buffered saline containing 10 µg/ml in concentration of an anti-mouse CD80 monoclonal antibody (hereinafter referred to as "antibody solution") was added to 4 wells, and 10 µg/ml in concentration of an anti-mouse CD86 monoclonal antibody solution to 4 wells, each at 1 ml, and incubated at 37°C for 8 hours or more.

Next, the polyNIP AAm-g-PP non-woven fabric treated with the antibody solution was washed twice with PBS of 37°C, and a 5×10⁵/ml CD80 hybridoma suspension suspended in RPMI-1640 medium (Sigma Aldrich Japan) was added at 1 ml to respective wells and incubated at 37°C for 45 minutes or 60 minutes.

After the incubation and subsequent washing of the polyNIP AAm-g-PP non-woven fabric twice with PBS of 37°C, methanol fixation and Giemsa staining were carried out, and the number of captured cells was counted by observing under a microscope.

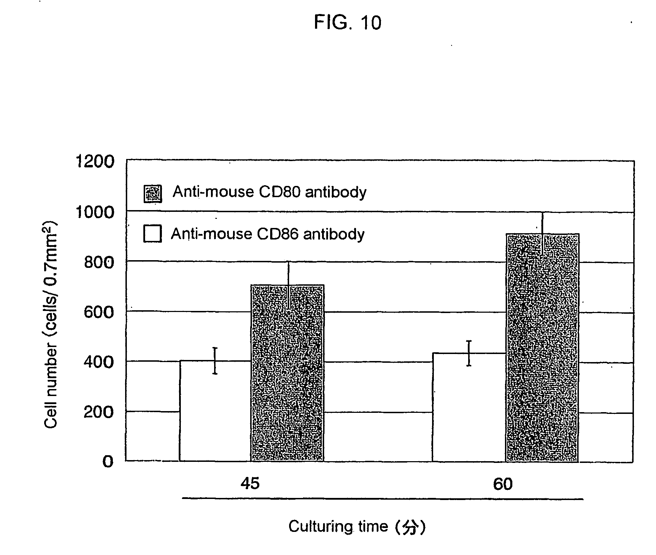

The cell capture accelerating effect was found in the polyNIP AAm-g-PP non-woven fabric incubated with the anti-mouse CD80 monoclonal antibody solution more significantly than the case of the anti-mouse CD86 monoclonal antibody solution (Fig. 10). In addition, more selective cell capturing effect was found when the incubation time was 60 minutes rather than 45 minutes.

### Cell separation test with polyNIP AAm-g-PP non-woven fabric

Using the polyNIP AAm-g-PP non-woven fabric, cell separation performance of this non-woven fabric was confirmed by carrying out a test in which one kind of cells (CD80 hybridoma) are recovered from a suspension of two kinds of cells (CD86 hybridoma and CD80 hybridoma).

### Materials and apparatuses:

- Non-woven fabric:: polyNIP AAm-modified non-woven fabric of 1.5 cm in diameter, 12 pieces
- Antibody concentration:: anti-CD80, 10 µg/ml (suspended in PBS)
- Cell suspension:: 5×10⁵/ml (CD80/CD86 = 50/50) suspended in a medium for cells
- Medium for cells:: RPMI-1640 medium (Sigma Aldrich Japan)
- Contact time of cells:: 45 minutes or 60 minutes (37°C)

### Test method:

(1) The polyNIP AAm-g-PP non-woven fabric was put into each well of a 24 well plate and held with a glass ring to prevent floating of the non-woven fabric.
(2) The antibody solution of anti-CD80 was dispensed at 0.5 ml and incubated at 37°C for 3 hours to bind the antibody to the polyNIP AAm-g-PP non-woven fabric.
(3) In order to wash away the excess antibody, the polyNIP AAm-g-PP non-woven fabric was washed twice with PBS of 37°C.
(4) The CD80 hybridoma and CD86 hybridoma were suspended in RPMI-1640 medium. The concentration was set to 5×10⁵/ml (CD80/CD86 = 50/50).
(5) This cell suspension was dispensed at 1 ml in wells and incubated at 37°C for 45 minutes.
(6) The polyNIP AAm-g-PP non-woven fabric was taken out and washed twice with PBS of 37°C in order to wash away the excess cells.
(7) In order to recover the captured cells, the polyNIP AAm-g-PP non-woven fabric was put into 10 ml of RPMI-1640 medium and exposed to 4°C for 15 minutes.
(8) The recovered cells were stained with fluorescence-labeled anti-CD80 and anti-CD86 antibodies.
(9) The stained cells were spread on a hemocytometer, and the recovery yield of the cells was calculated.
(10) The ratio of the CD80 hybridoma and CD80 hybridoma (the target cells) in the recovered cells was calculated by confocal laser microscope observation.

### Test results:

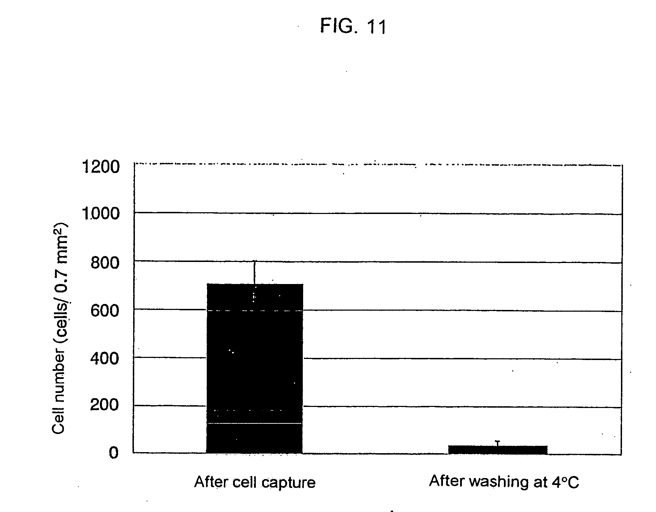

About 90% of the cells captured by the polyNIPAAm-g-PP non-woven fabric can be recovered (Fig. 11).

The recovered cells were CD80 hybridoma/CD86 hybridoma = about 70/30.

### Industrial Applicability

As has been described above, according to the present invention, the target cells can be separated and recovered conveniently and securely, in such a manner that damage is not inflicted on the cells. Accordingly, it is possible to apply the present invention to, for example, a case in which predetermined cells are separated and recovered in bio-tissue engineering, cell therapy and the like.

## Claims

1. A cell separation and recovery apparatus, which comprises:
a treatment part having a non-woven fabric which is bound with a polymer showing a hydrophobic nature at a temperature higher than a predetermined temperature and showing a hydrophilic nature at a temperature lower than the predetermined temperature and a physiologically active substance capable of binding to target cells, and
a liquid temperature controlling part for controlling liquid temperature of the treatment part,
wherein the cells captured on the non-woven fabric are released and recovered from the non-woven fabric by changing the liquid temperature of the treatment part around the prescribed temperature with the liquid temperature controlling part.

2. The cell separation and recovery apparatus according to claim 1, wherein the physiologically active substance is bound to the non-woven fabric via the polymer.

3. The cell separation and recovery apparatus according to claim 1, wherein the physiologically active substance is directly bound to the non-woven fabric.

4. The cell separation and recovery apparatus according to claim 1, wherein the physiologically active substance is bound to the non-woven fabric via a spacer.

5. The cell separation and recovery apparatus according to any one of claims 1 to 4, which is constituted so as to capture the cells on the non-woven fabric captures when the liquid temperature is lower than the predetermined temperature, and to release the captured cells from the non-woven fabric when the liquid temperature is higher than the predetermined temperature.

6. The cell separation and recovery apparatus according to any one of claims 1 to 4, which is constituted so as to capture the cells on the non-woven fabric when the liquid temperature is higher than the predetermined temperature, and to release the captured cells from the non-woven fabric when the liquid temperature is lower than the predetermined temperature.

7. The cell separation and recovery apparatus according to any one of claims 1 to 6, wherein the polymer is poly(N-isopropylacrylamide).

8. The cell separation and recovery apparatus according to any one of claims 1 to 6, wherein the physiologically active substance is at least one selected from the group consisting of an antigen, an antibody and a protein such as a fragment thereof.

9. A method for separating and recovering cells, which comprises:
bringing a liquid containing cells into contact with a non-woven fabric which is bound with a polymer showing a hydrophobic nature at a temperature higher than a predetermined temperature and showing a hydrophilic nature at a temperature lower than the predetermined temperature and a physiologically active substance capable of binding to target cells, to thereby capture target cells on the non-woven fabric for separating the cells,
releasing the captured cells from the non-woven fabric by changing the temperature of the non-woven fabric around the predetermined temperature, and
recovering the cells released from the non-woven fabric.

10. A method for separating and recovering cells, which comprises:
bringing a liquid containing cells into contact with the non-woven fabric in the treatment part using the separation and recovery apparatus according to any one of claims 1 to 8 to capture target cells on the non-woven fabric for separating the cells from the liquid,
releasing the captured cells from the non-woven fabric by changing the liquid temperature of the treatment part around the predetermined temperature, and
recovering the cells released from the non-woven fabric.
